# EUROPEAN PATENT APPLICATION

(11) **EP 3 270 337 A1**
(43) Date of publication of application: **17.01.2018**
(21) Application number: 17178630.4
(22) Date of filing: 29.06.2017
(51) Int. Cl.: G06Q 10/06, G06Q 50/22

(54) **METHOD AND SYSTEM FOR MANAGING ELECTRONIC INFORMED CONSENT PROCESS IN CLINICAL TRIALS**

(30) Priority: 15.07.2016 FI 20165591
(71) Applicant: CRF Box Oy, 00100 Helsinki (FI)
(72) Inventor: Järvisalo, Jari, 33400 Tampere (FI); Lindroos, Mika, 02450 Sundsberg (FI); Savilampi, Erkki, 02230 Espoo (FI)
(74) Representative: Papula Oy

(57) **Abstract**

A method and a system for managing an electronic informed consent process in a clinical trial are provided. The method includes tracking (704) time spent by a participant on reading content associated with an electronic informed consent process in a clinical trial. The time spent on reading the content is compared (706) with a pre-defined expected time for reading the content. A deviation in the time spent is detected (708) if difference between the time spent and the pre-defined expected time does not meet a time difference threshold. Furthermore, the method includes triggering (710) an action to mitigate impact of the deviation on the electronic informed consent process.

## Description

### FIELD OF THE INVENTION

The present application generally relates to informed consent in clinical trials, and more particularly to a method and system for managing electronic informed consent process in clinical trials.

### BACKGROUND OF THE INVENTION

An informed consent process is a process by which a participant voluntarily confirms his or her willingness to participate in a clinical trial after having been informed of all aspects of the clinical trial that are relevant to the participant's decision to participate in the clinical trial. The informed consent process is a regulated process and different jurisdictions have set forth different requirements for the informed consent process. Typically, information presented to the participant in the clinical trial, the process used for obtaining informed consent and a documentation of the informed consent process must meet these requirements among other applicable requirements.

Over the years, the highly regulated informed consent process has not seen much improvement to support better recruitment and retention of participants. Informed consent also remains as one of the most cited deficiencies by regulatory authorities and it is also associated with increased risk of delayed approvals in clinical trials.

Current solutions for informed consent are manual in nature and are inefficient. For example, a participant has to go through several paper-based documents in order to confirm willingness to participate in a clinical trial. These paper-based documents are lengthy and difficult for the participant to comprehend. Further, since the process is manual, there is no guarantee that the participant has indeed read the content of these documents.

In some conventional solutions, an attempt is made to make the informed consent process electronic. In such solutions, the participants are provided the content electronically and some tracking is done to determine the time consumed in different steps of the electronic informed consent process and expected time to achieve overall consent. The data related to time is then provided to a clinical trial investigator to meet regulatory requirements. However, mere tracking of time is not enough and still does not ensure if the participant has read the content or not. For example, tracked time may show that the participant took five seconds to read a three-page document, which in most cases is not enough to read a three-page document, and this tracked time is merely displayed to the clinical trial investigator without any effort to ensure that the participant has actually read the content.

### SUMMARY OF THE INVENTION

The following presents a simplified summary of the disclosure in order to provide a basic understanding to the reader. This summary is not an extensive overview of the disclosure and it does not identify key/critical elements or delineate the scope of the specification. Its sole purpose is to present a selection of concepts disclosed herein in a simplified form as a prelude to the more detailed description that is presented later.

In an embodiment, a method for managing an electronic informed consent process in a clinical trial is disclosed. The method includes tracking time spent by a participant on reading content associated with the electronic informed consent process. The time spent on reading the content is compared with a pre-defined expected time for reading the content. The method includes detecting a deviation if a difference between the time spent and the pre-defined expected time does not meet a time difference threshold. The method further includes triggering an action during the electronic informed consent process to mitigate impact of the deviation on the electronic informed consent process.

In an embodiment, a system for managing an electronic informed consent process in a clinical trial is disclosed. The system includes at least one processor and at least one memory comprising computer program code. The at least one memory and the computer program code configured to, with the at least one processor, cause the system to at least perform tracking of time spent by a participant on reading a section of an electronic informed consent form. The system is caused to compare the time spent on reading the content with a pre-defined expected time for reading the content. A deviation is detected if a difference between the time spent and the pre-defined expected time does not meet a time difference threshold. The system is further caused to trigger an action during the electronic informed consent process to mitigate impact of the deviation on the electronic informed consent process.

In an embodiment, another system for managing electronic informed consent process in a clinical trial is provided. The system includes a time spent tracker, a comparator, a deviation detector and an action triggering unit. The time spent tracker tracks time spent by a participant on reading a section of an electronic informed consent form. The comparator compares the time spent on reading the section with a pre-defined expected time for reading the section. The deviation detector detects a deviation if a difference between the time spent and the pre-defined expected time does not meet a time difference threshold. The action triggering unit triggers an action during the electronic informed consent process to mitigate impact of the deviation on the electronic informed consent process.

Many of the attendant features will be more readily appreciated, as the same becomes better understood by reference to the following detailed description considered in connection with the accompanying drawings.

### BRIEF DESCRIPTION OF THE DRAWINGS

The present description will be better understood from the following detailed description read in light of the following accompanying drawings, wherein:
FIG. 1 illustrates an example environment, where various embodiments of the present disclosure may be practiced;
FIG. 2 is a block diagram of a system configured to facilitate management of an electronic informed consent process, in accordance with an example embodiment;
FIG. 3 is a block diagram showing a plurality of modules for managing an electronic informed consent process, in accordance with an example embodiment;
FIG. 4 is a block diagram showing a plurality of modules for determining pre-defined expected time, in accordance with an example embodiment;
FIG. 5 is a block diagram showing a plurality of modules for determining pre-defined expected time, in accordance with another example embodiment;
FIG. 6 is a block diagram showing a plurality of modules for managing an electronic informed consent process, in accordance with another example embodiment;
FIG. 7 illustrates an example flow diagram of a method for managing an electronic informed consent process, in accordance with an example embodiment;
FIG. 8 illustrates an example flow diagram of a method for managing an electronic informed consent process, in accordance with another example embodiment;
FIG. 9 illustrates an example of an electronic device capable of implementing example embodiments described herein; and
FIGS. 10 to 12 depict user interfaces of an example electronic informed consent application displayed to a participant on an electronic device, in accordance with an example embodiment.

Like reference numerals are used to designate like parts in the accompanying drawings.

### DETAILED DESCRIPTION

In various embodiments, systems and methods for managing an electronic informed consent process are provided. The electronic informed consent process is a part of overall clinical trial process. The electronic informed consent process is performed to fulfill regulatory requirement of obtaining informed consent. The informed consent process is a process by which a participant (also referred to as "subject") voluntarily confirms his or her willingness to participate in a clinical trial after having been informed of all aspects of the clinical trial that are relevant to the participant's decision to participate in the clinical trial.

The participant accesses an electronic informed consent process application. The electronic informed consent process application provides an electronic informed consent form to the participant and is hosted by an electronic informed consent server (hereinafter referred to as "server"). The participant goes through the content of the electronic informed consent form. The content is divided into one or more sections, for example "overview of the clinical trial", "introduction of the informed consent process", "risks and benefits", "alternate treatments" and the like. A time spent by a participant in performing an activity, for example reading, on a section of an electronic informed consent form is tracked. The time may be tracked using web tracking or content tracking techniques. For example, the content includes a piece of analytics code, which tracks events performed by the participant and sends the events to the server. Some examples of the events include clicks, mouse hovering, selecting/unselecting options and the like, performed by the participant. In some embodiments, an eye-tracking application is present on device of the participant to track eyes of the participant and to send data to the server. The server receives the data and processes the data to draw meaningful information out of the data. It is noted that the server tracks the data in conjunction with the device of the participant and analyzes the data to determine time spent on the section of the content. The time spent is then compared against a pre-defined expected time for performing the activity. The pre-defined expected time for performing the activity is determined based on at least one of past recorded data for performing the activity on the section of the electronic informed consent form, or an average time estimate for performing the activity on the section of the electronic informed consent form. The past recorded data may be of same participant or different participants. In some embodiments, if the participant is a first time participant then the pre-defined expected time includes the average time estimate as the pre-defined expected time. In some scenarios, the pre-defined expected time is determined using one or more parameters associated with the content of the electronic informed consent form, the participant and/or the activity.

Further, a deviation in the time spent is detected if a difference between the time spent in performing the activity and the pre-defined expected time for performing the activity does not meet a time difference threshold. The time difference threshold is set at an optimal level to ensure that a minor deviation is ignored while a major deviation is detected. In an example embodiment, the time difference threshold may be associated with a minimum value and a maximum value. For example, the minimum value and the maximum value of the time difference threshold for reading a 'risk and benefits' section of the electronic informed consent form are three minutes and ten minutes, respectively. If the difference between the time spent in reading (i.e. performing an activity) and the pre-defined expected time for reading is between three and ten minutes, then no deviation is detected. However, if the difference between the time spent in reading and the pre-defined expected time for reading is less than three minutes or over ten minutes, then it may be deduced that the difference does not meet the time difference threshold, and, accordingly a deviation may be detected.

Based on detection of the deviation, an action is triggered during the electronic informed consent process to mitigate impact of the deviation on the electronic informed consent process. In an example scenario, an impact of deviation on the electronic informed consent process may be early termination of the electronic informed consent process by the participant. For example, a participant may have spent too much time in understanding a section and he/she may get frustrated on account of not understanding the content clearly. The participant in such a case may choose to terminate the electronic informed consent process. In some cases, the participant may choose to never return to participate in clinical trials, thereby hampering subject retention. Accordingly, an action is triggered during the electronic informed consent process to mitigate impact of the deviation on the electronic informed consent process. For example, a coordinator coordinating the clinical trial or the electronic informed consent process is alerted that a deviation has occurred. The term 'coordinator' as used herein may refer to any personnel entrusted with assisting the participant with the electronic informed consent process. The term 'coordinator' may also, in some scenarios, encompass more than one individual. For example, in facilities like hospitals or clinics, one or more nurses and/or other assistants may assist the participant during the consenting process. The term coordinator, in such scenarios, may refer to a single individual, like a doctor, overseeing the entire electronic informed consent process for various participants or to his/her assistants entrusted with assisting the individual participants during the course of their respective consenting process.

The alert message provided to the coordinator may include details of the participant, such as the participant location information, the participant identification information and the like. The participant location information may include, for example, information of the room number (or ward number) currently housing the participant within the facility, where the electronic informed consent process is being executed. The participant identification information may include personal details of the participant, such as name, participant id, contact details and the like. In some embodiments, the coordinator may initiate a chat conversation or a phone conversation with the participant upon receiving the alert message. In some example scenarios, the coordinator may physically meet up with the participant to assist the participant during the consenting process.

In some embodiments, a pop-up or alert message may also be shown on the device of the participant to indicate that the deviation has occurred and the participant may contact the coordinator. A communication channel is then established between the participant and the coordinator.

In various embodiments, tracking activities of the participant ensures that the participant is engaged in the content and has consumed the content. The real time triggering of the action enables the coordinator to solve problem of the participant and to save time of the participant. In addition, since the coordinator knows the section that the participant is consuming, the coordinator may anticipate questions and may better prepare himself/herself for solving the problems of the participant. The participant also feels secure because the corrective measures are taken during the electronic informed consent process. In addition, the overall electronic informed consent process becomes efficient as corrective measures are taken during the electronic informed consent process. In addition, the electronic informed consent process is interactive due to involvement of both the participant and the coordinator during the electronic informed consent process.

The various embodiments of the present disclosure are now described in detail using various figures.

It is noted that the detailed description provided below in connection with the appended drawings is intended as a description of the present examples and is not intended to represent the only forms in which the present example may be constructed or utilized. However, the same or equivalent functions and sequences may be accomplished by different examples.

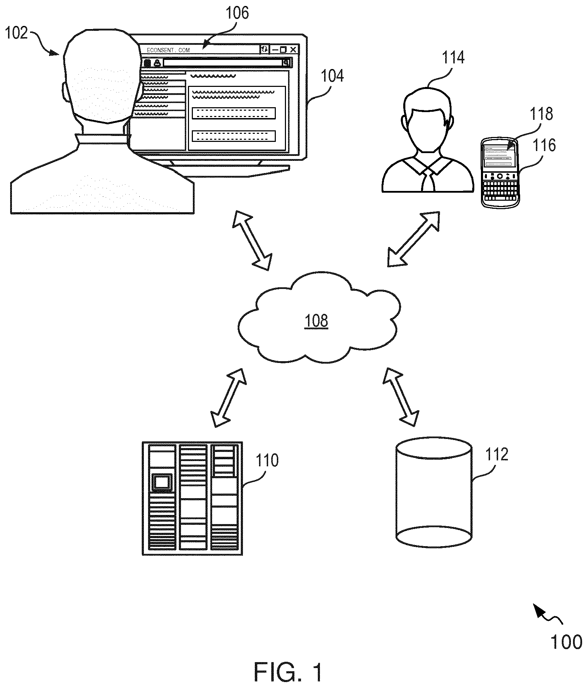

FIG. 1 illustrates an example environment 100, where various embodiments of the present disclosure may be practiced. An example representation of the environment 100 is shown depicting a network 108 that connects entities such as a plurality of users (e.g., users 102 and 114) and a server 110. The network 108 may be a centralized network or may comprise a plurality of sub-networks that may offer a direct communication between the entities or may offer indirect communication between the entities. Examples of the network 108 include wireless networks, wired networks, and combinations thereof. Some non-exhaustive examples of wireless networks may include wireless local area networks (WLANs), Bluetooth or Zigbee networks, cellular networks and the like. Some non-exhaustive examples of wired networks may include Local Area Networks (LANs), Ethernet, Fiber Optic networks and the like. An example of a combination of wired networks and wireless networks may include the Internet.

The environment 100 includes one or more participants that are associated with one or more electronic devices to communicate with other entities of the environment 100 via the network 108. For example, a participant 102 is connected to the network 108 via a device 104, and a coordinator 114 is connected to the network 108 via a device 116. The devices may be connected to the network 108 using, for example a private or a public network, a LAN, a wireless network, a Bluetooth based network, or any such type of network. It is noted that devices 104 and 116 may not be limited to a desktop computer and a mobile phone, respectively, as shown in the environment 100 and that the entities may connect to the network 108 using various devices. Examples of the devices include, but are not limited to, laptops, smartphones, tablets, smart watches, smart televisions, smart devices in homes, and other such systems having capability to participate in electronic informed consent process.

In some example embodiments, the server 110 may include one or more processing elements (e.g., computing systems, databases, etc.) to process the information received from various devices and to manage the electronic informed consent process.

In some example embodiments, the server 110 maintains an infrastructure for hosting applications, such as for example an electronic informed consent process application, an instance of which may be installed on the devices of the entities. The entities using the electronic informed consent process application may interact with the server 110, for example, they may provide data and receive data from the server 110. The functionalities of the server 110 may be embodied in form of cloud services and/or subscription services.

In one embodiment, the server 110 and one or more devices of the entities execute a method for managing an electronic informed consent process as described in present disclosure. In another embodiment, the server 110 receives one or more inputs from the one or more devices of the entities and performs an entire method as described in present disclosure. In yet another embodiment, the one or more devices of the entities perform entire method as described herein.

The device 104 is depicted to include an electronic informed consent process application 106. The electronic informed consent process application 106 is hereinafter referred to as application 106. The application 106 enables the participant 102 to provide data and receive data from the server 110. Similarly, the device 116 is depicted to include the electronic informed consent process application 118. The electronic informed consent process application 118 is hereinafter referred to as application 118. In one embodiment, the application 106 may differ from the application 118, as different versions of the applications may be provisioned based on different devices and the device form-factors, or based on different type on entities. In such scenario, the workflow offered may differ based on subscriptions rights of the entities and type of entities. For example, the participant 102 will see a workflow related to filling of electronic informed consent form and signing it while a coordinator 114 will see workflows related to tracking of the electronic informed consent form, creating the form, taking corrective measures during the electronic informed consent form, and managing the electronic informed consent form.

In another embodiment, the application 106 may be similar to the application 118 and the applications may correspond to mere different instances of same application running on different devices.

In an embodiment, the coordinator 114 or any other form creating entity may create the electronic informed consent form as part of the electronic informed consent process and make the electronic informed consent form available for consumption by participants via electronic informed consent process applications, such as the applications 106 and 118. While creating the electronic informed consent form, the coordinator 114 or the form creating entity may designate sections of the electronic informed consent form for which tracking is desired. In some embodiments, the designation may be performed by embedding a piece of tracking code in corresponding sections of the electronic informed consent form. The electronic informed consent form may then be made available for consumption via the server 110. In some embodiments, the server 110 may also facilitate the creation of the electronic informed consent form.

In at least one example embodiment, the electronic informed consent form is divided into one or more sections. The sections may be created based on parameters, such as for example type of content, nature of content and so on and so forth. Some non-exhaustive examples of the type of content may include text-based content, audio-video or multimedia content, image content and the like. Some non-exhaustive examples of nature of the content may include introducing the electronic informed consent process to the participant, informing the participant of risks and benefits of the clinical trial, clarifying queries and the like.

In an embodiment, the participant 102 may access the electronic informed consent form by accessing the application 106 via the device 104. Examples of the application 106 include, but are not limited to, a standalone software application, a standalone electronic informed consent form or an electronic portal accessed via a browser. In at least one example embodiment, the application 106 may be configured to be tracking enabled and cause tracking of activities performed on the application 106 or the electronic informed consent form. Examples of the activities include, but are not limited to, reading, gazing, filling, deleting, selecting an option, unselecting an option, navigating, resuming and the like.

In one embodiment, the tracking code is embedded in the application 106 while in another embodiment, a reference to the tracking code is embedded in the application 106 and the tracking code indicating what sections of the electronic informed consent form are to be tracked may be fetched from the server 110. The time spent in performing an activity on a section of the electronic informed consent form is then tracked. The tracking may be performed per activity, per section, per participant and/or based on any other granularity as desired by the form creating entity or a form managing entity or any other entity involved with the electronic informed consent form, and hereinafter collectively referred to as "the form managing entity".

The data collected as part of tracking is sent to the server 110 by the device 106 in batches or as a continuous stream. The server 110 then accesses the data and processes the data to draw meaningful information. In one embodiment, the tracking includes a combination of steps performed at the device 106 and the server 110. In another embodiment, the tracking refers to the steps performed at the device 106. In yet another embodiment, the tracking refers to the analysis performed by the server 110.

It is to be appreciated that any other technology for tracking may also be used. For example, the tracking may be performed by applications outside the electronic informed consent process application and the data may be reported to the server 110.

In an embodiment, the server 110 compares the time spent for performing an activity on a section of the content with a pre-defined expected time for performing the activity on the section. It is noted that the basis for comparing the time spent and the pre-defined expected time may be chosen to be same, for example, per section, per activity and the like. In some embodiments, the base for comparison includes exactly a same portion of process flow in the electronic informed consent process. For example, the process flow may be combination of one or more sections and hence, may have one pre-defined expected time for the combined sections. The process flow may be defined as any activity or activities performed on any section or sections of the electronic informed consent form.

In at least one example embodiment, the pre-defined expected time is determined by the server 110 based on at least one of: (1) past recorded data of performing the activity by the participant during one or more prior electronic informed consent processes; (2) an average time estimate for performing the activity determined from time spent in performing the activity by prior participants of the electronic informed consent process; and (3) one or more parameters related to the content, the activity or the participant. In one embodiment the numbering (1) to (3) above does not indicate any priority order for determining the pre-defined expected time. In another embodiment, determining the pre-defined expected time is performed in the order of (1) to (3), wherein first using (1), if available, and if not then (2) if available, and if not then (3) . In a further embodiment determining the pre-defined expected time may be performed as a combination of any of definitions (1) to (3).

Some non-exhaustive examples of the parameters related to the content include number of sections in the content, number of questions in each section, number of words or sentences in the section, number of bullet points in the section, difficulty of the question, wording of the question, order of question, response categories, speed vs. accuracy instructions, learning effects, and the like. Some non-exhaustive examples of parameters related to the participant may include age or cognitive ability of the participant, the participant's familiarity with the content, health of the participant, education of the participant, participant's tendency to acquiescence and the like. Some non-exhaustive examples of parameters related to the activity may include time pressure, current mood/attention of participant and the like.

The server 110 includes several modules for determining the pre-defined expected time as will be explained in detail later. In some embodiments, the pre-defined expected time may also be fed or modified by the form managing entity. The server 110 further detects a deviation if the difference between the time spent and the pre-defined expected time does not meet a time difference threshold. For example, if the difference is beyond, i.e. more than or less than, certain desired level then it is considered to be not meeting the time difference threshold. The time difference threshold may be set and modified by the form managing entity or may be set and modified based on past data.

Based on detection of the deviation, the server 110 then triggers an action during the electronic informed consent process to mitigate the impact of the deviation. In one embodiment, the server 110 performs a check if sufficient time or a time beyond a minimum-time-left-threshold is left in the electronic informed consent process. If yes, then the server 110 triggers the action. Examples of the action include, but are not limited to, providing a corrective measure to mitigate impact of the deviation. In some embodiments, the mitigation is done by alerting the participant 102 or the coordinator 114 or both of the occurrence of deviation. The alert may be in any form, such as for example a message notification, a flash notification, a flash notification combined with a beeping sound, an email and the like. As explained with reference to FIG. 1, the notification alerting the coordinator may include participant location information, participant identification information and the like. Additionally, the notification may also include probable reason for deviation and in some cases, a link to a self-help section for enabling the coordinator to assist the participant in response to the detected deviation. In addition, a channel of communication is established between the participant 102 and the coordinator 114 to enable them to interact and mitigate the impact of deviation by providing corrective measures. The coordinator 114 may coach or help the participant 102, and the participant 102 may ask queries to the coordinator 114. The channel of communication may be via the electronic informed consent process application or any other application.

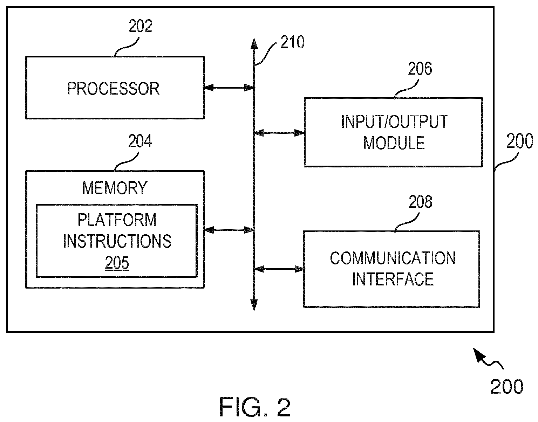

The environment 100 also includes a database 112 in which various data for access by the server 110, the device 106 or the device 116 is stored. The data may include time spent, prior history of the participant, or any other data. The database 112 may be accessed directly or via the network 108. The management of the electronic informed consent process is further explained with reference to FIG. 2.

FIG. 2 is a block diagram of a system 200 configured to facilitate management of an electronic informed consent process, in accordance with an example embodiment. The system 200 includes at least one processor such as a processor 202 and at least one memory such as a memory 204. The system 200 also includes an input/output module 206 and a communication interface 208. The system 200 may be included within the server 110 explained with reference to FIG. 1, or in some embodiments, the system 200 may embody the server 110. In some embodiments, one or more components of the system 200 may be deployed in any user device such as the device 104 of the participant or the device 116 of the coordinator.

Although the system 200 is depicted to include only one processor 202, the system 200 may include more number of processors therein. In an embodiment, the memory 204 is capable of storing platform instructions 205, where the platform instructions 205 are machine executable instructions associated with managing electronic informed consent process. Further, the processor 202 is capable of executing the stored platform instructions 205. In an embodiment, the processor 202 may be embodied as a multi-core processor, a single core processor, or a combination of one or more multi-core processors and one or more single core processors. For example, the processor 202 may be embodied as one or more of various processing devices, such as a coprocessor, a microprocessor, a controller, a digital signal processor (DSP), a processing circuitry with or without an accompanying DSP, or various other processing devices including integrated circuits such as, for example, an application specific integrated circuit (ASIC), a field programmable gate array (FPGA), a microcontroller unit (MCU), a hardware accelerator, a special-purpose computer chip, or the like. In an embodiment, the processor 202 may be configured to execute hard-coded functionality. In an embodiment, the processor 202 is embodied as an executor of software instructions, wherein the instructions may specifically configure the processor 202 to perform the algorithms and/or operations described herein when the instructions are executed.

The memory 204 may be embodied as one or more volatile memory devices, one or more non-volatile memory devices, and/or a combination of one or more volatile memory devices and non-volatile memory devices. For example, the memory 204 may be embodied as magnetic storage devices (such as hard disk drives, floppy disks, magnetic tapes, etc.), optical magnetic storage devices (e.g., magneto-optical disks), CD-ROM (compact disc read only memory), CD-R (compact disc recordable), CD-R/W (compact disc rewritable), DVD (Digital Versatile Disc), BD (BLU-RAY® Disc), and semiconductor memories (such as mask ROM, PROM (programmable ROM), EPROM (erasable PROM), flash ROM, RAM (random access memory), etc.).

The input/output module 206 (hereinafter referred to as 'I/O module 206') is configured to facilitate provisioning of an output and/or receiving an input. The I/O module 206 is configured to be in communication with the processor 202 and the memory 204. Examples of the I/O module 206 include, but are not limited to, an input interface and/or an output interface. Examples of the input interface may include, but are not limited to, a keyboard, a mouse, a joystick, a keypad, a touch screen, soft keys, a microphone, and the like. Examples of the output interface may include, but are not limited to, a display such as a light emitting diode display, a thin-film transistor (TFT) display, a liquid crystal display, an active-matrix organic light-emitting diode (AMOLED) display, a microphone, a speaker, a ringer, a vibrator, and the like. In an example embodiment, the processor 202 may include I/O circuitry configured to control at least some functions of one or more elements of the I/O module 206, such as, for example, a speaker, a microphone, a display, and/or the like. The processor 202 and/or the I/O circuitry may be configured to control one or more functions of the one or more elements of the I/O module 206 through computer program instructions, for example, software and/or firmware, stored on a memory, for example, the memory 204, and/or the like, accessible to the processor 202.

In an embodiment, the I/O module 206 may be configured to provide a user interface (UI) configured to provide options or any other display to a user of the system 200. In addition, the I/O module 206 may be integrated with mechanisms configured to receive inputs from the user of the system 200.

The communication interface 208 may enable the system 200 to communicate with other devices, such as the devices of the participants and the coordinators of the clinical trials over various types of networks such as the network 108 as explained with reference to FIG. 1. For example, the communication interface 208 may provision an electronic informed consent application, such as the application 106 or the application 118, to the participant. The communication interface 208 may also facilitate provision of notifications to coordinators of the electronic informed consent process and/or clinical trials upon detection of deviation in time spent on an activity. The communication interface 208 may facilitate reception of tracking data, hand/eye movement actions etc. from the devices of the participants.

In an embodiment, various components of the system 200, such as the processor 202, the memory 204, the I/O module 206 and the communication interface 208 are configured to communicate with each other via or through a centralized circuit system 210. The centralized circuit system 210 may be various devices configured to, among other things, provide or enable communication between the components (202 - 208) of the system 200. In certain embodiments, the centralized circuit system 210 may be a central printed circuit board (PCB) such as a motherboard, a main board, a system board, or a logic board. The centralized circuit system 210 may also, or alternatively, include other printed circuit assemblies (PCAs) or communication channel media.

The system 200 as illustrated and hereinafter described is merely illustrative of a system that could benefit from embodiments of the invention and, therefore, should not be taken to limit the scope of the invention. It is noted that the system 200 may include fewer or more components than those depicted in FIG. 2.

As explained above, the system 200 may embody the server 110. In another embodiment, the system 200 may be a standalone component in a remote machine connected to a communication network (such as the network 108 explained with reference to FIG. 1) and capable of executing a set of instructions (sequential and/or otherwise). Moreover, the system 200 may be implemented as a centralized system, or, alternatively, the various components of the system 200 may be deployed in a distributed manner while being operatively coupled to each other. In an embodiment, one or more functionalities of the system 200 may also be embodied as a client within devices, such as the device 106 of the participant 102 or the device 116 of the coordinator 114. In another embodiment, the system 200 may be a central system that is shared by or accessible to each of such devices.

The management of the electronic informed consent process by the system 200 is explained hereinafter with reference to various modules implementing functionalities of the processor 202 of the system 200. It is to be appreciated that the system components, modules or units as described herein can be implemented using one processor or multiple processors or using one system described in FIG. 2 or using multiple such systems. It is noted that, in some embodiments, the processor 202 may be substituted by a combination of individual modules, such that the combination of individual modules perform similar functions as that by the processor 202.

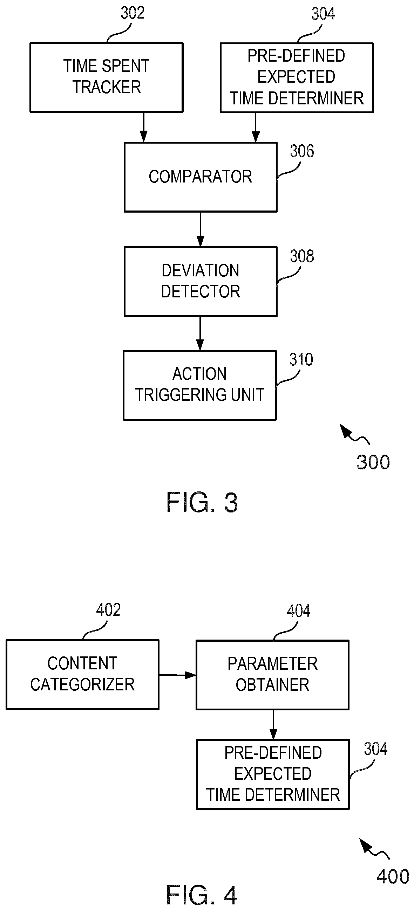

FIG. 3 is a block diagram 300 showing a plurality of modules for managing an electronic informed consent process, in accordance with an example embodiment. In an embodiment, the plurality of modules shown in the block diagram 300 may, in effect, configure the processor 202 of the system 200 for performing the methods as described herein. The plurality of modules depicted in the block diagram 300 includes a time spent tracker 302, a pre-defined expected time determiner 304, a comparator 306, a deviation detector 308 and an action triggering unit 310.

The time spent tracker 302 is configured to track time spent in performing an activity on content associated with the electronic informed consent process. In at least one example embodiment, the content includes one or more sections of the electronic informed consent form. The time spent can be tracked using any existing technique or tracking code. The tracking can be performed per activity, per section, per participant or based on any other granularity as desired by the form managing entity. The data collected (such as for example, time stamps) as part of tracking is processed by the time spent tracker 302 to determine the time spent on the activity.

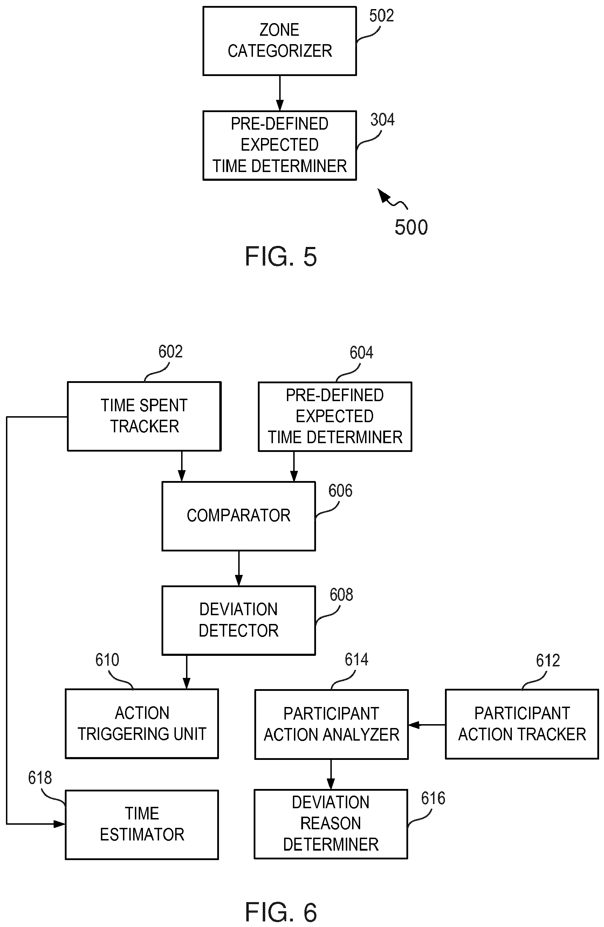

The pre-defined expected time determiner 304 is configured to determine the pre-defined expected time for performing the activity based on at least one: (1) past recorded data of performing the activity by the participant during one or more prior electronic informed consent processes; (2) an average time estimate for performing the activity determined from time spent in performing the activity by prior participants of the electronic informed consent process; and (3) one or more parameters related to the section and the participant. In one embodiment the numbering (1) to (3) above does not indicate any priority order for determining the pre-defined expected time. In another embodiment, determining the pre-defined expected time is performed in the order of (1) to (3), wherein first using (1), if available, and if not then (2) if available, and if not then (3). In a further embodiment determining the pre-defined expected time may be performed as a combination of any of definitions (1) to (3). The determination of the pre-defined expected time may be performed as explained with reference to FIG. 1. The average time estimate can be fed or modified by the form managing entity or can be determined by the server 110 as will be described in FIG. 4 or FIG. 5.

The comparator 306 is configured to compare the time spent with the pre-defined expected time. The deviation detector 308 is configured to detect a deviation if difference between the time spent and the pre-defined expected time does not meet the time difference threshold. The action triggering unit 310 is configured to trigger an action, i.e. a corrective measure, during the electronic informed consent process to mitigate the impact of the deviation. In some embodiments, the term 'mitigating the impact of deviation' implies resolving the concern of the participant, for example by interacting with the participant, such that a remaining portion of the electronic informed consent process is completed without any deviation.

FIG. 4 is a block diagram 400 showing a plurality of modules for determining pre-defined expected time, in accordance with an example embodiment. In an embodiment, the modules depicted in the block diagram 400 include a content categorizer 402, a parameter obtainer 404 and the pre-defined expected time determiner 304. The modules depicted in the block diagram 400 may be a part of the processor 202 of the system 200 explained with reference to FIG. 2. Alternatively, the various functionalities executed by the processor 202 for determining the pre-defined expected time are depicted to be embodied as modules in the block diagram 400.

The content categorizer 402 is configured to categorize content associated with the electronic informed consent process into one or more sections or one or more process flows or to any other level of granularity as desired by the form managing entity. The content categorizer 402 divides the content into a base that can be used for comparison. The base may include any level of granularity, i.e. sub-sections to a combination of one or more sections, sub-activity to a combination of one or more activities, or a combination of one or more activities for one or more sections or any other possible combination. In one embodiment, a category or the base of comparison may be defined as any profile or flow step of the electronic informed consent process that can be created from metadata or data associated with the electronic informed consent process.

The parameter obtainer 404 is configured to obtain one or more parameters associated with each category created by the content categorizer 402. The parameters may be based on at least one of the activity, the section of the content, and the participant. Examples of the parameters include, but are not limited to, type of activity, nature of the section, number of words in the section, number of questions in the section, number of figures in the section, age of participant, typing speed of participant, typing speed of participant for a particular section, or any other parameter that can be associated with any possible category.

For each parameter for each category, the data is fetched from the database 112 (explained with reference to FIG. 1) by the pre-defined expected time determiner 304. The database 112 is configured to store information related to content of several electronic informed consent forms, such as for example number of sections, words, images, questions etc. The database 112 is also configured to store activity data for several participants. Examples of data fetched from the database 112 include, but are not limited to, data related to the current electronic informed consent process/form as well data of the participant such as profile, name, address, age, gender, type of device (such as a mobile phone, laptop etc.) used by the participant or type of input (for example, keyboard, mouse, touch screen etc.) provided by the participant and the like. In some embodiments, data fetched from the database 112 may also include data, such as whether the section includes one or more tick mark questions or one or more questions eliciting subjective response and the like. In one embodiment, the fetched data from the database may include values corresponding to each parameter. The pre-defined expected time determiner 304 may process the data to determine the pre-defined expected time. In some embodiments, different weights may be assigned to different parameters and weighted average techniques may be used to arrive at the pre-defined expected time. In at least one example embodiment, the determined pre-defined expected time may be in form of a numerical value (for example, 'x' seconds or minutes, where x is any non-zero positive integer). It is noted that the pre-defined expected time may also be a non-numerical value. The pre-defined expected time is then saved in the memory 204 of the system 200 for access by other modules of the processor 202. It is noted that the pre-defined expected time is different for different activities and/or different sections of the content. In some embodiments, the pre-defined expected time can be fed or modified by the form managing entity.

FIG. 5 is a block diagram 500 showing a plurality of modules for determining pre-defined expected time, in accordance with another example embodiment. In an embodiment, the modules depicted in the block diagram 500 include a zone categorizer 502 and the pre-defined expected time determiner 304. The modules depicted in the block diagram 500 may be a part of the processor 202 of the system 200 explained with reference to FIG. 2. Alternatively, the various functionalities executed by the processor 202 for determining the pre-defined expected time are depicted to be embodied as modules in the block diagram 500.

The zone categorizer 502 is configured to categorize content portions or sections into one or more zones based on the nature of respective content. For example, a content portion or a section highlighting risks and benefits of the clinical trial may require more careful reading as compared to a section introducing the process to the participant. In an example embodiment, a section requesting the participant to input personal details may be categorized as a 'comfort zone' by the zone categorizer 502, whereas another section introducing the electronic informed consent process may be categorized as a 'semi-comfort zone'. The section related to risks and benefits of the clinical trial or the section related to alternate treatments may be categorized by the zone categorizer 502 as a 'zone of discontent' as such sections may require more careful reading and selection by the participant. However, it is to be appreciated that based on preferences of the form managing entity the zone classification of different sections may vary.

The pre-defined expected time determiner 304 is configured to determine different pre-defined expected time for each of such zones. In an embodiment, historic estimates of traversing these zones by several participants may be taken into account while determining pre-defined expected times for each zone. The pre-defined expected time can also be fed manually or modified manually by the form managing entity.

It is to be appreciated that the pre-defined expected time can be a number, a range, or can take any other form that is representative of time. In some embodiments, and as shown in FIG. 5, the zone of the content section is one parameter based on which the pre-defined expected time may be determined.

Additionally, for each zone there can be a comfort zone speed of the participant. The comfort zone speed is defined as speed of the participant at which the participant performs a desired activity on the section under given circumstances. In an embodiment, the speed at which a participant fills in personal details, may be used to calculate the participant's "comfort zone average" which further can be used as a base measure when determining whether the participant is progressing at expected/average speed. It is noted that each of the 'zones' may have their own expected rate of speed at which an average participant may proceed and complete the section.

FIG. 6 is a block diagram 600 showing a plurality of modules for managing an electronic informed consent process, in accordance with another example embodiment. In an embodiment, the plurality of modules shown in the block diagram 600 may, in effect, configure the processor 202 of the system 200 for performing the methods as described herein. The plurality of modules depicted in the block diagram 600 includes a time spent tracker 602, a pre-defined expected time determiner 604, a comparator 606, a deviation detector 608, an action triggering unit 610, a participation action tracker 612, a participation action analyzer 614, a deviation reason determiner 616 and a time estimator 618. The time spent tracker 602, the pre-defined expected time determiner 604, the comparator 606, the deviation detector 608 and the action triggering unit 610 are configured to be substantially similar (i.e. perform similar functions) to the time spent tracker 302, the pre-defined expected time determiner 304, the comparator 306, the deviation detector 308 and the action triggering unit 310 of FIG. 3, respectively, and are not explained again herein.

The participant action tracker 612 is configured to track or monitor actions of the participant. For example, the participant action tracker 612 may track hand movements, inputs (clicks, mouse hovering, selections etc.), gestures, and/or eye movements while the participant is performing one or more activities on the content associated with the electronic informed consent process. The participant action analyzer 614 is configured to analyze the monitored actions of the participant to draw meaningful inferences. Finally, from these meaningful inferences the deviation reason determiner 616 is configured to deduce a reason for the deviation. For example, if the time spent on a section and the pre-defined expected time exceeds the time difference threshold for that section and the monitored data shows that the participant switched back and forth in the content then that is deduced as the reason for deviation and the coordinator can contact the participant to take corrective action.

The time estimator 608 is configured to estimate at least one of time left to complete remaining electronic informed consent process, time to reach a next stage in electronic informed consent process and total time to complete the electronic informed consent process based, at least partially, on the time spent in performing the activity. For example, an average speed of traversal through sections may be computed by the time estimator 618 and used to determine how much time the participant may take to complete the remaining portion of the electronic informed consent form. In some embodiments, for determining the time left to complete remaining portion(s) of electronic informed consent process, the time estimator 618 may sum up the time spent so far and the pre-defined expected time for remaining granular segments.

In at least one embodiment, a participant is assigned to a group from among a plurality of groups based on participation data of the participant in prior electronic informed consent processes. In an embodiment, the pre-defined expected time for various activities/sections is different for different groups. The groups can be formed based on parameters associated with the participant and in some embodiments, are analogous to the granular segment. In some embodiments, based on input from the time estimator 618, if it is determined that performance of the participant in a granular segment does not conform or is different than an expected performance from the participant, then a group of the participant can be changed. In an example embodiment, the expected performance is defined in terms of time, i.e. if the difference between the time spent and the pre-defined expected time does not meet a threshold, then the expected performance is said to be different. The threshold can be selected based on prior data or can be manually fed or modified and can be set a little higher than the time difference threshold.

It is to be appreciated that although the present disclosure is described in light of corrective measures offered during the electronic informed consent process, the tracked data can be used later, i.e. post the electronic informed consent process, to draw meaningful inferences for the form managing entity to improve the form and/or remove sections or activities that take too long time.

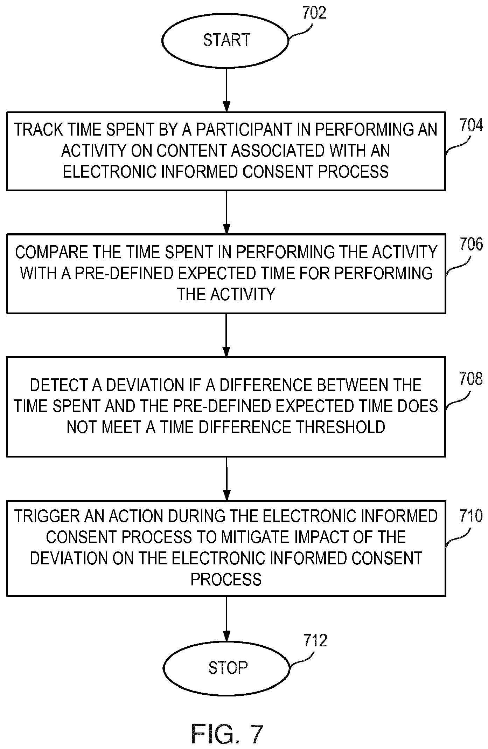

FIG. 7 illustrates an example flow diagram of a method 700 for managing an electronic informed consent process, in accordance with an example embodiment. The method 700 can be performed by the system 200 in combination with the participant's and/or coordinator devices, or by a combination of the server 110 and any of the participant's and/or coordinator's devices. The method 700 starts at 702.

At 704 of the method 700, time spent by a participant in performing an activity on content associated with an electronic informed consent process is tracked. In one embodiment, the content includes one or more sections of an electronic informed consent form. In another embodiment, the content can include a granular content section (for example, an individual question or a sentence within a section) or even broader content section (for example, a process flow or one or more sections) as described earlier.

At 706 of the method 700, the time spent in performing the activity is compared with a pre-defined expected time for performing the activity. The pre-defined expected time for performing the activity may be determined based on at least one of past recorded data for performing the activity on the section of the electronic informed consent form, or an average time estimate for performing the activity on the section of the electronic informed consent form. The past recorded data may be of same participant or different participants. In some embodiments, if the participant is a first time participant then the pre-defined expected time includes the average time estimate as the pre-defined expected time. In some scenarios, the pre-defined expected time is determined using one or more parameters associated with the participant, the activity and/or the content of the electronic informed consent form.

At 708 of the method 700, a deviation is detected if a difference between the time spent and the pre-defined expected time does not meet a time difference threshold.

At 710 of the method 700, an action is triggered during the electronic informed consent process to mitigate impact of deviation on the electronic informed consent process. The triggering includes providing a notification alerting a coordinator and, in some scenarios, establishing a channel of communication between the coordinator and the participant for providing corrective measures. The coordinator may be any personnel entrusted with assisting the participant with the electronic informed consent process as explained with reference to FIG. 1. The notification provided to the coordinator may include details of the participant, such as the participant location information, the participant identification information and the like. In some embodiments, the coordinator may initiate a chat conversation or a phone conversation with the participant upon receiving the notification. In some example scenarios, the coordinator may physically meet up with the participant to assist the participant during the consenting process. In one embodiment, the participant is also alerted regarding deviation and availability of the corrective measure. The participant may choose to accept or decline the offer for corrective measure. If it is declined then no channel of communication is established.

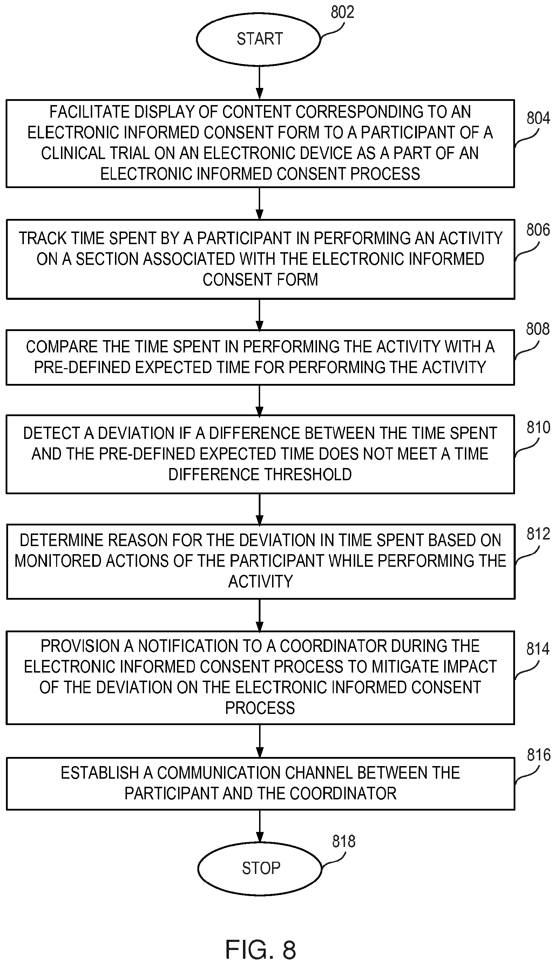

The method 700 stops at 710. Another method for managing of an electronic informed consent process is explained with reference to FIG. 8.

FIG. 8 illustrates an example flow diagram of a method 800 for managing an electronic informed consent process, in accordance with another example embodiment. The method 800 can be performed by the system 200 in combination with the participants' devices, or by a combination of the server 110 and any of the participants' devices. The method 800 starts at 802.

At 804 of the method 800, display of content corresponding to an electronic informed consent form to a participant of a clinical trial on an electronic device is facilitated as part of an electronic informed consent process. The content corresponding to the electronic informed consent form includes several sections.

At 806 of the method 800, time spent by a participant in performing an activity on a section associated with the electronic informed consent form is tracked.

At 808 of the method 800, the time spent in performing the activity is compared with a pre-defined expected time for performing the activity.

At 810 of the method 800, a deviation is detected if a difference between the time spent and the pre-defined expected time does not meet a time difference threshold.

At 812, a reason for the deviation in time spent is determined. The deviation in the time spent may be determined by monitoring hand movements and or eye movement (using hand/eye tracking technology) of the participant.

At 814 of the method 800, a notification is provisioned to the coordinator during the electronic informed consent process to mitigate impact of deviation on the electronic informed consent process. The notification includes the reason for deviation. In some embodiments, the notification may also include participation location information, participation identification information and a link to a self-help section for enabling the coordinator to assist the participant in response to the detected deviation.

At 816 of the method 800, a communication channel, such a chat channel is established between the participant and the coordinator.

The method 800 stops at 818.

The disclosed methods 700 and 800 may be implemented using software including computer-executable instructions stored on one or more computer-readable media (e.g., non-transitory computer-readable media, such as one or more optical media discs, volatile memory components (e.g., DRAM or SRAM), or nonvolatile memory or storage components (e.g., hard drives or solid-state nonvolatile memory components, such as Flash memory components) and executed on a computer (e.g., any suitable computer or image processor embedded in a device, such as a laptop computer, net book, web book, tablet computing device, smart phone, or other mobile computing device). Such software may be executed, for example, on a single local computer or in a network environment (e.g., via the Internet, a wide-area network, a local-area network, a remote web-based server, a client-server network (such as a cloud computing network), or other such network) using one or more network computers. Additionally, any of the intermediate or final data created and used during implementation of the disclosed methods or systems may also be stored on one or more computer-readable media (e.g., non-transitory computer-readable media) and are considered to be within the scope of the disclosed technology. Furthermore, any of the software-based embodiments may be uploaded, downloaded, or remotely accessed through a suitable communication means. Such suitable communication means include, for example, the Internet, the World Wide Web, an intranet, software applications, cable (including fiber optic cable), magnetic communications, electromagnetic communications (including RF, microwave, and infrared communications), electronic communications, or other such communication means.

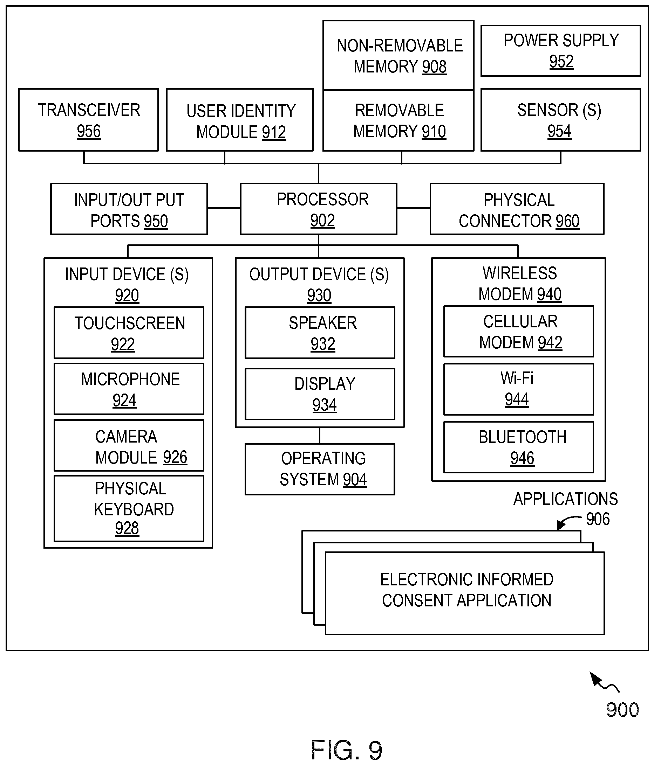

Referring now to FIG. 9, a schematic block diagram of a electronic device 900 is shown that is capable of implementing embodiments of techniques for managing electronic informed consent process as described herein. It should be understood that the electronic device 900 as illustrated and hereinafter described is merely illustrative of one type of device and should not be taken to limit the scope of the embodiments. As such, it should be appreciated that at least some of the components described below in connection with the electronic device 900 may be optional and thus in an example embodiment may include more, less or different components than those described in connection with the example embodiment of FIG. 9. As such, among other examples, the electronic device 900 could be any of device from among fixed electronic devices, such as desktop computers and electronic kiosks, to mobile electronic devices, such as for example, personal digital assistants (PDAs), mobile televisions, cellular phones, tablet computers, laptops, mobile computers or any combination of the aforementioned, and other types of communication or multimedia devices.

In at least one example embodiment, the electronic device 900 may be a participant device. For example, a participant may carry a personal electronic device to a facility, such as for example a hospital or a clinic, which is entrusted with the execution of the electronic informed consent process for various probable participants. In such a scenario, a coordinator of the electronic informed consent process, such as the coordinator explained with reference to FIG. 1, may execute, either personally or through other assistants, the loading of an electronic informed consent application (see applications 906) onto the personal electronic device of the participant. In some scenarios, the electronic informed consent application may be accessed through the web browser installed on the personal electronic device of the participant or downloaded through an online application store onto the personal electronic device.

Alternatively, in some embodiments, the facility may provide participants with electronic devices pre-installed with electronic informed consent applications. In at least one embodiment, the electronic device 900 may correspond to a kiosk device pre-installed with electronic informed consent application.

It is noted that the computer program code corresponding to the electronic informed consent application installed in the electronic device 900 may not only be configured to display one or more user interfaces associated with electronic informed consent form, but also cause the electronic device to perform various functionalities associated with managing the electronic informed consent process, such as for example, tracking time spent by a participant in performing an activity on an section associated with the electronic informed consent form; comparing the time spent in performing the activity with a pre-defined expected time for performing the activity; detecting a deviation if a difference between the time spent and the pre-defined expected time does not meet a time difference threshold; and triggering an action during the electronic informed consent process to mitigate impact of the deviation on the electronic informed consent process. The management of the electronic informed consent process may be performed by the electronic device 900 as explained with reference to FIGS. 1 to 8 and is not explained again herein.

The illustrated electronic device 900 includes a controller or a processor 902 (e.g., a signal processor, microprocessor, ASIC, or other control and processing logic circuitry) for performing tasks such as signal coding, data processing, image processing, input/output processing, power control, and/or other functions. In at least one example embodiment, the processor 902 may include modules depicted in the block diagram 600 of FIG. 6 for enabling the execution of the electronic informed consent process. An operating system 904 controls the allocation and usage of the components of the electronic device 900 and support for one or more applications programs (see, applications 906), such as the electronic informed consent application, that implements one or more of the innovative features described herein. In addition to electronic informed consent application, the applications 906 may include common mobile computing applications (e.g., telephony applications, email applications, calendars, contact managers, web browsers, messaging applications) or any other computing application.

The illustrated electronic device 900 includes one or more memory components, for example, a non-removable memory 908 and/or removable memory 910. The non-removable memory 908 can include RAM, ROM, flash memory, a hard disk, or other well-known memory storage technologies. The removable memory 910 can include flash memory, smart cards, or a Subscriber Identity Module (SIM). The one or more memory components can be used for storing data and/or code for running the operating system 904 and the applications 906. Examples of data can include web pages, text, images, sound files, image data, video data, or other data sets to be sent to and/or received from one or more network servers or other devices via one or more wired or wireless networks. The electronic device 900 may further include a user identity module (UIM) 912. The UIM 912 may be a memory device having a processor built in. The UIM 912 may include, for example, a SIM, a universal integrated circuit card (UICC), a universal subscriber identity module (USIM), a removable user identity module (R-UIM), or any other smart card. The UIM 912 typically stores information elements related to a mobile subscriber. The UIM 912 in form of the SIM card is well known in Global System for Mobile Communications (GSM) communication systems, Code Division Multiple Access (CDMA) systems, or with third-generation (3G) wireless communication protocols such as Universal Mobile Telecommunications System (UMTS), CDMA9000, wideband CDMA (WCDMA) and time division-synchronous CDMA (TD-SCDMA).

The electronic device 900 can support one or more input devices 920 and one or more output devices 930. Examples of the input devices 920 may include, but are not limited to, a touch screen 922 (e.g., capable of capturing finger tap inputs, finger gesture inputs, multi-finger tap inputs, multi-finger gesture inputs, or keystroke inputs from a virtual keyboard or keypad), a microphone 924 (e.g., capable of capturing voice input), a camera module 926 (e.g., capable of capturing still picture images and/or video image frames) and a physical keyboard 928. Examples of the output devices 930 may include, but are not limited to a speaker 932 and a display 934. Other possible output devices (not shown) can include piezoelectric or other haptic output devices. Some devices can serve more than one input/output function. For example, the touch screen 922 and the display 934 can be combined into a single input/output device.

In an embodiment, the camera module 926 may include a digital camera capable of facilitating tracking of hand movements and/or eye movements of the participant. In some implementations, the camera module 926 may include two or more cameras, for example, a front camera and a rear camera positioned on two sides of the electronic device 900. As such, the camera module 926 includes all hardware, such as a lens or other optical component(s), and software for capturing images and/or creating a video stream from a captured video. Alternatively, the camera module 926 may include the hardware needed to view the video, while a memory device of the electronic device 900 stores instructions for execution by the processor 902 in the form of software to create a video stream from a captured video. In an example embodiment, the camera module 926 may further include a processing element such as a co-processor, which assists the processor 902 in processing image frame data and an encoder and/or decoder for compressing and/or decompressing image data. In an embodiment, the camera module 926 may provide live image data (viewfinder image data) to the display 934.

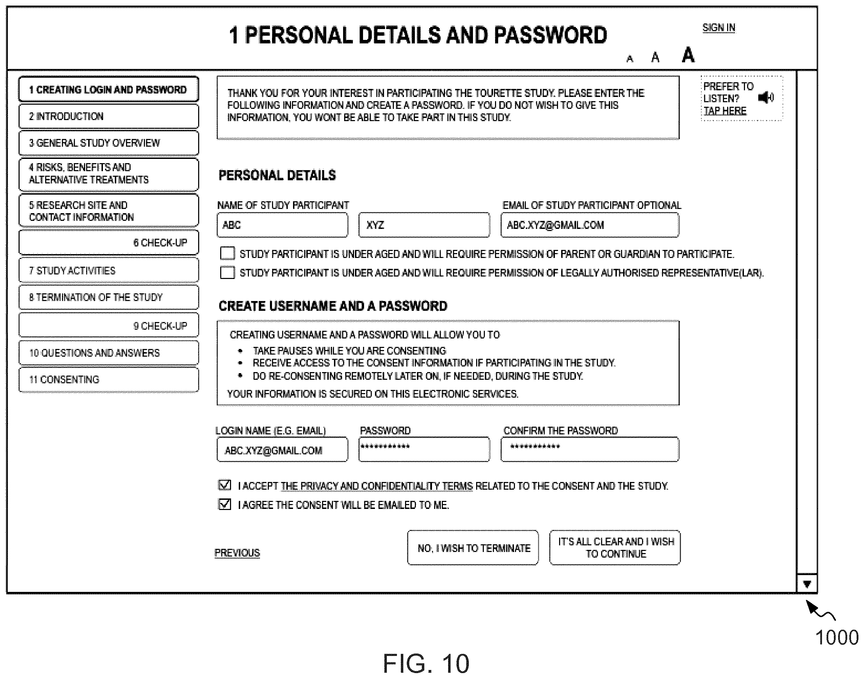

The display 934 is configured to display content, such as one or more UIs associated with an electronic informed consent form. The UIs associated with the electronic informed consent form are explained in detail with reference to FIGS. 10 to 12.

A wireless modem 940 can be coupled to one or more antennas (not shown in FIG. 9) and can support two-way communications between the processor 902 and external devices, as is well understood in the art. For example, the communication may include provisioning notifications to the coordinator, establishing a chat link between the participant and the coordinator and the like. The wireless modem 940 is shown generically and can include, for example, a cellular modem 942 for communicating at long range with the mobile communication network, a Wi-Fi-compatible modem 944 for communicating at short range with an external Bluetooth-equipped device or a local wireless data network or router, and/or a Bluetooth-compatible modem 946. The wireless modem 940 is typically configured for communication with one or more cellular networks, such as a GSM network for data and voice communications within a single cellular network, between cellular networks, or between the mobile device and a public switched telephone network (PSTN).

The electronic device 900 can further include one or more input/output ports 950, a power supply 952, one or more sensors 954 for example, an accelerometer, a gyroscope, a compass, or an infrared proximity sensor for detecting the orientation or motion of the electronic device 900, a transceiver 956 (for wirelessly transmitting analog or digital signals) and/or a physical connector 960, which can be a USB port, IEEE 1394 (FireWire) port, and/or RS-232 port. The illustrated components are not required or all-inclusive, as any of the components shown can be deleted and other components can be added.

FIGS. 10 to 12 depict user interfaces of an example electronic informed consent application displayed to a participant on an electronic device, such as the electronic device 900, in accordance with an example embodiment. The electronic informed consent application is depicted to show several tabs, such as '1. Creating Login and Password', '2. Introduction', '3. General Study Overview', '4. Risks, Benefits and Alternate Treatments', '5. Research Site and Contact Information' '6. Check up', '7. Study Activities', '8. Termination of the Study', '9. Check-up', '10. Questions and Answers', and 'Consenting'. It is noted that the tabs of the electronic informed consent application are depicted herein for illustration purposes and that the electronic informed consent application may not be limited to such a representation.

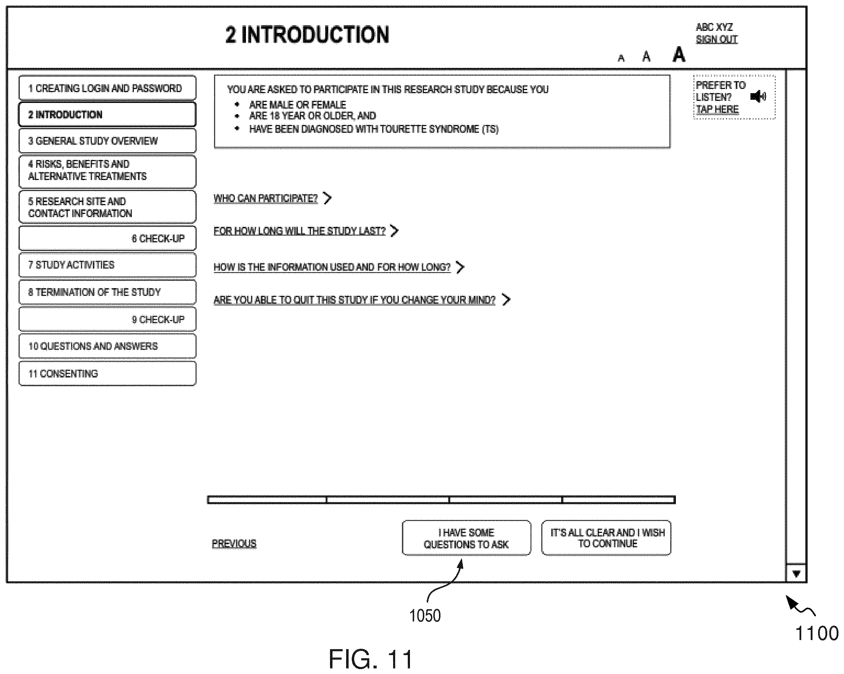

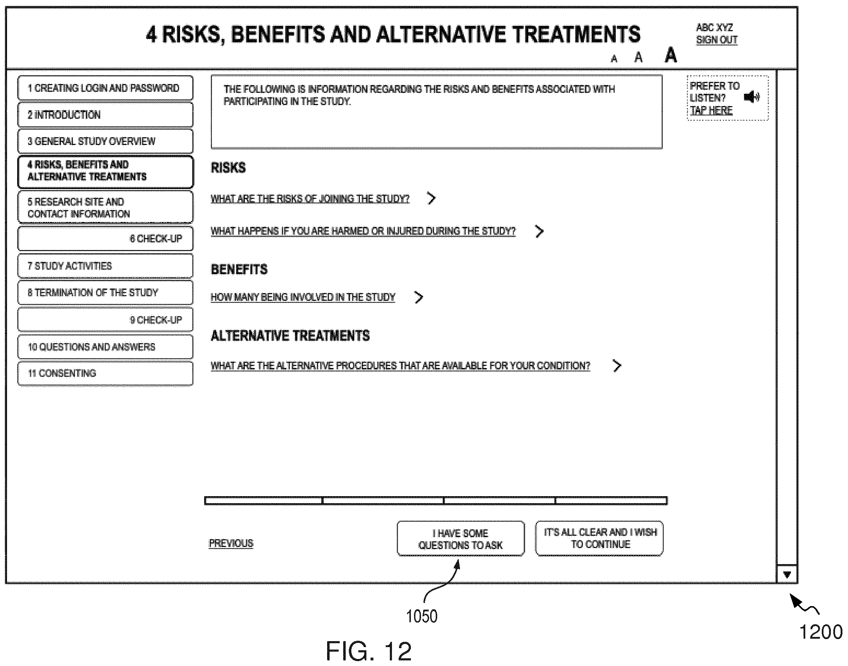

Each tab is associated with a corresponding user interface, such as a UI 1000 associated with the tab '1. Creating Login and Password' depicted in FIG. 10, a UI 1100 associated with tab '2. Introduction' depicted in FIG. 11 and a UI 1200 associated with tab '4. Risks, Benefits and Alternate Treatments' depicted in FIG. 12. The content associated with the various tabs of the electronic informed consent application together configures the content of the electronic informed consent process. In some embodiments, the content of the various tabs may in effect configure the electronic informed consent form, which the participant signs after traversing through the entire content in order to confirm his or her willingness to participate in the clinical trial. In some embodiments, content corresponding to each tab may be construed as a 'section' on which various activities, such as reading, provisioning a selection input, etc. may be performed. The time spent in performing each activity on each section (or on the overall content) may be associated with pre-defined expected times as explained with reference to FIGS. 1 to 9. Deviations in time spent by the participants from the respective pre-defined expected times may be detected and reported to coordinator of the electronic informed consent process.

As explained above, the content portions or the sections may be categorized into zones, such as for example, the section related to creating login and password may be categorized into 'comfort zone', the section related to introduction may be categorized into 'semi-comfort zone' and the section related to 'risks, benefits and alternate treatments' may be categorized into 'zone of discontent'. Further, pre-defined expected times for performing activities in each zone may be determined and actual time spent in performing those activities by participants may be compared to detect deviations. The deviations may be reported to a coordinator, in some embodiments, using a notification. In some embodiments, notification may be sent to the coordinator possibly including reference to the step/page where the deviance occurred or was detected, and possibly an iteration of the possible reason for which the deviation might have occurred, like "participant repeatedly switched back and forth between help and this page - could mean that the user failed to understand the question", wherein this information would help the coordinator to prepare themselves with the expected questions by the participant. In addition, a link to a related help section may be provided to the coordinator for facilitation of quick help to the participant. It is noted that the participant may also initiate interaction with the coordinator at any stage during the electronic informed consent process. For example, the participant may click on option 1050 displaying text 'I have questions to ask' on the UIs 1100 and 1200, at any stage during the electronic informed consent process to initiate interaction with the coordinator.

Various example embodiments offer, among other benefits, techniques for managing an electronic informed consent process in clinical trials. The methods and systems disclosed herein overcome several drawbacks of conventional mechanisms thereby supporting better recruitment and retention of participants. The electronic informed consent process as suggested herein is interactive due to involvement of both the participant and the coordinator during the electronic informed consent process.

In various embodiments, tracking activities of the participant ensures that the participant is engaged in the content and has consumed the content. The real time triggering of the action enables the coordinator to solve problem of the participant and to save time of the participant. In addition, since the coordinator knows the section that the participant is consuming, the coordinator may anticipate questions and may better prepare him/her for solving the problems of the participant. The participant also feels secure because the corrective measures are taken during the electronic informed consent process. In addition, the overall electronic informed consent process becomes efficient as corrective measures are taken during the electronic informed consent process.

It is noted that various example embodiments as described herein may be implemented in a wide variety of devices, network configurations and applications.

Computer executable instructions may be provided using any computer-readable media that is accessible by computing based device. Computer-readable media may include, for example, computer storage media such as memory and communications media. Computer storage media, such as memory, includes volatile and non-volatile, removable and non-removable media implemented in any method or technology for storage of information such as computer readable instructions, data structures, program modules or the like. Computer storage media includes, but is not limited to, RAM, ROM, EPROM, EEPROM, flash memory or other memory technology, CD-ROM, digital versatile disks (DVD) or other optical storage, magnetic cassettes, magnetic tape, magnetic disk storage or other magnetic storage devices, or any other non-transmission medium that may be used to store information for access by a computing device. In contrast, communication media may embody computer readable instructions, data structures, program modules, or the like in a modulated data signal, such as a carrier wave, or other transport mechanism. As defined herein, computer storage media does not include communication media. Therefore, a computer storage medium should not be interpreted to be a propagating signal per se. Although the computer storage media is shown within the computing-based device it will be appreciated that the storage may be distributed or located remotely and accessed via a network or other communication link, for example by using communication interface.

The methods described herein may be performed by software in machine readable form on a tangible storage medium e.g. in the form of a computer program comprising computer program code means adapted to perform all the operations of any of the methods described herein when the program is run on a computer and where the computer program may be embodied on a computer readable medium. Examples of tangible storage media include computer storage devices such as disks, thumb drives, memory etc. The software can be suitable for execution on a parallel processor or a serial processor such that the method operations may be carried out in any suitable order, or simultaneously.

Alternatively, or in addition, the functionality described herein (such as the electronic informed consent form managing instructions) can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), Graphics Processing Units (GPUs) . For example, some or all of the device functionality or method sequences may be performed by one or more hardware logic components.

The embodiments illustrated and described herein as well as embodiments not specifically described herein but within the scope of aspects of the invention constitute exemplary system means for managing the electronic informed consent process. For example, the elements illustrated and described with reference to FIGS. 1 and 11, when configured, under control of the processor 202 and program code in the memory 204 to perform the operations illustrated and described with reference to FIGS. 3, 4, 5, 6, 7 and 8, constitute an exemplary electronic informed consent processing application means for tracking time spent by a participant in performing an activity on content associated with the electronic informed consent process, comparing the time spent in performing the activity is compared with a pre-defined expected time for performing the activity, detecting a deviation if a difference between the time spent and the pre-defined expected time does not meet a time difference threshold, and triggering an action during the electronic informed consent process to mitigate impact of the deviation on the electronic informed consent process

The benefits and advantages described above may relate to one embodiment or may relate to several embodiments. The embodiments are not limited to those that solve any or all of the stated problems or those that have any or all of the stated benefits and advantages.

The operations of the methods described herein may be carried out in any suitable order, or simultaneously where appropriate. Additionally, individual blocks may be added or deleted from any of the methods without departing from the spirit and scope of the subject matter described herein. Aspects of any of the examples described above may be combined with aspects of any of the other examples described to form further examples without losing the effect sought.

The above description is given by way of example only and various modifications may be made by those skilled in the art. The above specification, examples and data provide a complete description of the structure and use of exemplary embodiments. Although various embodiments have been described above with a certain degree of particularity, or with reference to one or more individual embodiments, those skilled in the art could make numerous alterations to the disclosed embodiments without departing from the spirit or scope of this specification.

## Claims

1. A method for managing an electronic informed consent process in a clinical trial, the method comprising:
tracking (704, 806), by a processor, time spent by a participant on reading content associated with the electronic informed consent process;
comparing (706, 808), by the processor, the time spent on reading the content with a pre-defined expected time for reading said content;
detecting (708, 810), by the processor, a deviation if a difference between the time spent and the pre-defined expected time does not meet a time difference threshold; and
triggering (710; 814-816), by the processor, an action during the electronic informed consent process to mitigate impact of the deviation on the electronic informed consent process.

2. The method of claim 1, further comprising:
determining, by the processor, the pre-defined expected time for reading the content is based on at least one of:
past recorded data of reading the content by the participant during one or more prior electronic informed consent processes;
an average time estimate for reading the content determined from time spent in reading the content by prior participants of the electronic informed consent process; and
one or more parameters related to the content, the participant and reading the content.

3. The method of claim 1 or 2, further comprising:
categorizing content portions associated with the content into a plurality of zones based on nature of respective content portions, wherein the pre-defined expected time for reading the content is, at least in part, determined based on the categorization of the content portions into the plurality of zones.

4. The method of any of claims 1 - 3, further comprising:
assigning the participant, by the processor, to a group based on participation data of the participant in one or more prior electronic informed consent processes, wherein the pre-defined expected time for reading the content is, at least in part, determined based on the group assigned to the participant.

5. The method of claim 4, further comprising:
changing the group of the participant, by the processor, if a performance of the participant during the electronic informed consent process is different than an expected performance from the participant.

6. The method of any of claims 1 - 5, further comprising:
monitoring actions performed by the participant while reading the content; and
determining a reason for the deviation from the monitored actions.

7. The method of any of claims 1 - 6, further comprising:
estimating, by the processor, at least one of time left to complete remaining electronic informed consent process, time to reach a next stage in the electronic informed consent process and a total time to complete the electronic informed consent process based, at least in part, on the time spent on reading the content.

8. The method of any of claims 1 -7, wherein the content comprises at least one of:
one or more sections of an electronic informed consent form; and
one or more questions of the electronic informed consent form.

9. The method of any of claims 1 - 8, wherein triggering the action comprises:
providing a notification to a coordinator of the electronic informed consent process for reporting the deviation.

10. The method of claim 9, further comprising:
establishing a communication between the participant and the coordinator.

11. The method of claim 9, wherein the notification comprises at least one of:
a participant location information;
a participant identification information;
a reason for the deviation; and
a link to a self-help section for enabling the coordinator to assist the participant in response to the detected deviation.

12. A system (200) for managing an electronic informed consent process in a clinical trial, the system comprising:
at least one processor (202); and
at least one memory (204) comprising computer program code (205), the at least one memory (204) and the computer program code (205) configured to, with the at least one processor (202), cause the system (200) to at least perform:
track time spent by a participant on reading a section of an electronic informed consent form;
compare the time spent on reading the section with a pre-defined expected time for reading the section;
detect a deviation if a difference between the time spent and the pre-defined expected time does not meet a time difference threshold; and
trigger an action during the electronic informed consent process to mitigate impact of the deviation on the electronic informed consent process.

13. The system of claim 12, wherein the system is further caused to determine the pre-defined expected time for reading the section based on at least one of:
past recorded data of reading a section by the participant during one or more prior electronic informed consent processes;
an average time estimate for reading the section determined from time spent on reading the section by prior participants of the electronic informed consent process; and
one or more parameters related to the section, the participant and the reading.

14. The system of claim 12 or 13, wherein the system is further caused, at least in part to:
categorize the section of the electronic informed consent form into a zone from among a plurality of zones based on nature of content associated with the section, wherein the pre-defined expected time for reading the section of the electronic informed consent form is, at least in part, determined based on the zone.

15. The system of any of claims 12 - 14, wherein the system is further caused, at least in part to:
assign the participant to a group based on participation data of the participant in one or more prior electronic informed consent processes, wherein the pre-defined expected time for reading the section of the electronic informed consent form is, at least in part, determined based on the group assigned to the participant.

16. The system of any of claims 12 - 15, wherein the system is further caused, at least in part to:
monitor actions performed by the participant while reading the section; and
determine a reason for the deviation from the monitored actions.

17. The system of any of claims 12 - 16, wherein the system is further caused, at least in part to:
provide a notification to a coordinator of the electronic informed consent process for reporting the deviation.

18. The system of claim 17, wherein the system is further caused, at least in part to:
establish a communication between the participant and the coordinator.

19. The system of claim 17, wherein the notification comprises at least one of:
a participant location information;
a participant identification information;
a reason for the deviation; and
a link to a self-help section for enabling the coordinator to assist the participant in response to the detected deviation.

20. A system for managing an electronic informed consent process in a clinical trial, the system comprising:
a time spent tracker (302) for tracking time spent by a participant in reading a section of an electronic informed consent form;
a comparator (306) for comparing the time spent on reading the section with a pre-defined expected time for reading the section;
a deviation detector (308) for detecting a deviation if a difference between the time spent and the pre-defined expected time does not meet a time difference threshold; and
an action triggering unit (310) for triggering an action during the electronic informed consent process to mitigate impact of the deviation on the electronic informed consent process.

21. The system of claim 20, wherein the system further comprises:
a pre-defined expected time determiner for determining the pre-defined expected time for reading the section based on at least one of:
past recorded data of reading a section by the participant during one or more prior electronic informed consent processes;
an average time estimate for reading the section determined from time spent on reading the section by prior participants of the electronic informed consent process; and
one or more parameters related to the section, the participant and the reading.

22. The system of claim 20, wherein the system further comprises:
a participant tracker for monitoring actions performed by the participant while reading the section of the electronic informed consent form; and
a deviation reason determiner for determining a reason for the deviation from the monitored actions.

23. An electronic device (900) comprising:
a display (934);
at least one processor (902); and
at least one memory (908, 910) comprising computer program code corresponding to an electronic informed consent application (906) associated with an electronic informed consent process in a clinical trial, the at least one memory (908, 910) and the computer program code configured to, with the at least one processor (902), cause the electronic device (900) to at least perform:
display one or more user interfaces corresponding to an electronic informed consent form;
track time spent by a participant on reading a section of the electronic informed consent form;
compare the time spent on reading the section with a pre-defined expected time for reading the section;
detect a deviation if a difference between the time spent and the pre-defined expected time does not meet a time difference threshold; and
trigger an action during the electronic informed consent process to mitigate impact of the deviation on the electronic informed consent process.

24. The electronic device of claim 23, wherein the electronic device is further caused to determine the pre-defined expected time for reading the section based on at least one of:
past recorded data of reading a section by the participant during one or more prior electronic informed consent processes;
an average time estimate for reading the section determined from time spent on reading the section by prior participants of the electronic informed consent process; and
one or more parameters related to the section, the participant and the reading.

25. The electronic device of claim 23 or 24, wherein the electronic device is further caused, at least in part to:
categorize the section of the electronic informed consent form into a zone from among a plurality of zones based on nature of content associated with the section, wherein the pre-defined expected time for reading the section of the electronic informed consent form is, at least in part, determined based on the zone.

26. The electronic device of any of claims 23 - 25, wherein the electronic device is further caused, at least in part to:
assign the participant to a group based on participation data of the participant in one or more prior electronic informed consent processes, wherein the pre-defined expected time for reading the section of the electronic informed consent form is, at least in part, determined based on the group assigned to the participant.

27. The electronic device of any of claims 23 - 26, wherein the electronic device is further caused, at least in part to:
monitor actions performed by the participant while reading the section; and
determine a reason for the deviation from the monitored actions.

28. The electronic device of any of claims 23 - 27, wherein the electronic device is further caused, at least in part to:
provide a notification to a coordinator of the electronic informed consent process for reporting the deviation.

29. The electronic device of claim 28, wherein the system is further caused, at least in part to:
establish a communication between the participant and the coordinator.

30. The electronic device of claim 28, wherein the notification comprises at least one of:
a participant location information;
a participant identification information;
a reason for the deviation; and
a link to a self-help section for enabling the coordinator to assist the participant in response to the detected deviation.

31. The electronic device of any of the claims 23 - 30, wherein the electronic device corresponds to a participant device.

32. The electronic device of any of the claims 23 - 30, wherein the electronic device is one of a mobile phone, a smartphone, a tablet computer, an electronic kiosk, a laptop computer, a wearable device, a desktop computer and a personal digital assistant.

33. A computer program product comprising at least one computer-readable storage medium, the computer-readable storage medium comprising a set of instructions, which, when executed by one or more processors (902), cause an electronic device (900) to at least perform:
track time spent by a participant on reading a section of an electronic informed consent form displayed on the electronic device (900) during an electronic informed consent process;
compare the time spent on reading the section with a pre-defined expected time for reading the section;
detect a deviation if a difference between the time spent and the pre-defined expected time does not meet a time difference threshold; and
trigger an action during the electronic informed consent process to mitigate impact of the deviation on the electronic informed consent process.

34. The computer program product of claim 33, wherein the electronic device is further caused, at least in part to:
determine the pre-defined expected time for the reading the section is based on at least one of:
past recorded data of reading a section by the participant during one or more prior electronic informed consent processes;
an average time estimate for reading the section determined from time spent in reading the section by prior participants of the electronic informed consent process; and
one or more parameters related to the content, the participant and the reading.

35. The computer program product of claim 33 or 34, wherein the electronic device is further caused, at least in part to:
categorize content portions associated with the content into a plurality of zones based on nature of respective content portions, wherein the pre-defined expected time for reading the section is, at least in part, determined based on the categorization of the content portions into the plurality of zones.

36. The computer program product of any of claims 33 - 35, wherein the electronic device is further caused, at least in part to:
assign the participant to a group based on participation data of the participant in one or more prior electronic informed consent processes, wherein the pre-defined expected time for reading the section is, at least in part, determined based on the group assigned to the participant.

37. The computer program product of claim 36, wherein the electronic device is further caused, at least in part to:
change the group of the participant if a performance of the participant during the electronic informed consent process is different than an expected performance from the participant.

38. The computer program product of any of claims 33 - 37, wherein the electronic device is further caused, at least in part to:
monitor actions performed by the participant while reading the section; and
determine a reason for the deviation from the monitored actions.

39. The computer program product of any of claims 33 - 38, wherein the electronic device is further caused, at least in part to:
estimate at least one of time left to complete remaining electronic informed consent process, time to reach a next stage in the electronic informed consent process and a total time to complete the electronic informed consent process based, at least in part, on the time spent on reading the section.

40. The computer program product of any of claims 33 - 39, wherein for triggering the action, the electronic device is further caused, at least in part to:
provide a notification to a coordinator of the electronic informed consent process for reporting the deviation.

41. The computer program product of claim 40, wherein the electronic device is further caused, at least in part to:
establish a communication between the participant and the coordinator.

42. The computer program product of claim 40, wherein the notification comprises at least one of:
a participant location information;
a participant identification information;
a reason for the deviation; and
a link to a self-help section for enabling the coordinator to assist the participant in response to the detected deviation.
